**Europäisches Patentamt**

**European Patent Office**

**Office européen des brevets**

(19)

(11) Veröffentlichungsnummer : **0 021 129**
**B2**

(12) **NEUE EUROPÄISCHE PATENTSCHRIFT**

(45) Veröffentlichungstag der neuen Patentschrift :
07.09.88

(21) Anmeldenummer : 80103046.1

(22) Anmeldetag : 31.05.80

(51) Int. Cl.⁴ : **A 61 K  9/16**, A 61 K 37/547,
**C 12 N  9/94**

(54) **Pankreatin-Pellets, Verfahren zu deren Herstellung und diese Pellets enthaltende Arzneimittel.**

(30) Priorität : 08.06.79 DE 2923279

(43) Veröffentlichungstag der Anmeldung :
07.01.81 Patentblatt 81/01

(45) Bekanntmachung des Hinweises auf die Patenterteilung : 01.02.84 Patentblatt 84/05

(45) Bekanntmachung des Hinweises auf die Entscheidung
über den Einspruch : 07.09.88 Patentblatt 88/36

(84) Benannte Vertragsstaaten :
AT BE CH DE FR GB IT LI LU NL SE

(56) Entgegenhaltungen :
DE-A- 2 035 739
DE-A- 2 135 025
DE-A- 2 512 746
DE-A- 2 626 109
DE-B- 1 617 363
US-A- 3 922 339
CHEMICAL ABSTRACTS, Band 89, Heft 18, 30. Oktober 1978, Seite 370, Zusammenfassung 152739y,
Columbus, Ohio, USA
H.Sucker et al., Pharmazeutische Technologie, 1978,
S. 333÷404
Pharmazeutische Zeitung, Apotheker Zeitung, 1975,
S. 680ff
HP-MCP Technical Information, No. H-5, March 1975,
No. H-6, August 1975 und No. H-10, March 1976
"The Yakugyo Jiho", 11.03.77
L. EMRARDT, Acta Pharm. Technol. Suppl., 1976. 125-
140
RÖMPPS Chemie Lexikon, 8. Aufl., S. 2169
BASF, Techn.-Merkblatt,RKollidon-Marken, Dez. 1979
Die Akte enthält technische Angaben, die nach dem
Eingang der Anmeldung eingereicht wurden und die
nicht in dieser Patentschrift enthalten sind.

(73) Patentinhaber : Kali-Chemie Pharma GmbH
Hans-Böckler-Allee 20 Postfach 220
D-3000 Hannover 1 (DE)

(72) Erfinder : Wischniewski, Martin, Dr.rer.nat
Kattowitzer Weg 24
D-3057 Neustadt a.Rbge (DE)
Erfinder : Feicho, Lutz
Paracelsusweg 17
D-3057 Neustadt a.Rbge (DE)
Erfinder : Fischer, Gerhard, Dr.rer.nat.
Grabbenweg 59
D-3000 Hannover 72 (DE)
Erfinder : Peschke, Günter Dr.rer.nat. Dipl.-Chem.
Hartestrasse 5
D-3000 Hannover 72 (DE)

(74) Vertreter : Lauer, Dieter, Dr.
c/o Kali-Chemie Aktiengesellschaft Postfach 220
D-3000 Hannover 1 (DE)

**Beschreibung**

Die Erfindung betrifft ein Verfahren zur Herstellung von Pankreatin-Pellets, sowie gemäß dem Verfahren hergestellte Pankreatin-Pellets und derartige Pellets enthaltende Arzneimittel.

Die Herstellung pharmazeutischer Pellets erfolgt hauptsächlich nach zwei verschiedenen Technologien. Entweder wird in einer geeigneten Anlage wie z. B. Pelletierteiller, Wirbelschichtanlage oder Dragierkessel aus einem sogenannten Saatkorn durch wechselweises Auftragen von flüssigen und festen Stoffen auf das rollende bzw. wirbelnde Saatkorn eine mehr oder weniger sphärische partikelform aufgebaut (Aufbaugranulation), oder es werden Zuckerglobuli vorgelegt, auf deren Oberfläche der Wirkstoff fixiert wird.

Beide Methoden sind für die Herstellung von Pankreatin-Pellets kaum anwendbar.

Bei der Aufbaugranulation wird zum Aufbau der Pellets sehr viel flüssige Phase, bestehend aus Wasser und/oder diversen Lösungsmitteln benötigt. Zur Herstellung von Pankreatin-Pellets scheidet Wasser wegen der äußerst feuchtigkeitsempfindlichen Pankreas-Enzyme völlig aus und auch die meisten bei der Aufbaugranulation verwendeten Lösungsmittel sind mehr oder minder enzymschädigend. Wird ein enzymfreundliches Lösungsmittel wie Isopropanol oder Aceton verwendet, so ist für die Herstellung der Pellets ein mehrfaches der Gewichtsmenge an Lösungsmitteln erforderlich, was generell unrentabel und problematisch im Hinblick auf Immissionsschutzbestimmungen ist. Häufig sind derart hergestellte Pellets mechanisch wenig stabil.

Nach der 2. Methode können in der Regel nur relativ geringe Wirkstoffmengen auf der Oberfläche der vorgelegten Zuckerglobuli untergebracht werden, was ebenfalls nur unter Einsatz relativ großer Mengen einer flüssigen Phase möglich ist. Da Pankreatin-Präparate jedoch in Einzeldosen von erheblich mehr als 200 mg gegeben werden müssen, scheidet auch diese Methode für die Herstellung von Pankreatin-Pellets aus.

Aus der DE-OS 2 626 109 ist bekannt, ein Gemisch, das pankreatische Enzyme, Bindemittel und Sprengmittel zwingend enthält und vorteilhafterweise noch einen Stabilisator enthält, unter Zusatz von inertem Lösungsmittel (z. B. Isopropanol oder Aceton) herzustellen. Dieses Gemisch wird dann zu Segmenten stranggepreßt, die abgerundet, getrocknet und magensaftresisten beschichtet werden. Selbst in der vorteilhaften Variante des Beispiels 1 der DE-OS 2 626 109, in der ein Bindemittel verwendet wird, sowie mit Isopropanol das beste Lösungsmittel eingesetzt wird, werden im Darmsaft nur noch 62 % der ursprünglichen Aktivität an Lipase, dem Leitenzym des Pankreatins, freigesetzt, d. h. über 1/3 der ursprünglichen Aktivität geht verloren.

Demgemäß besteht die Aufgabe der Erfindung darin, ein Verfahren zur Herstellung von Pankreatin-Pellets zur Verfügung zu stellen, das die Nachteile bestehender Verfahren vermeidet.

Gegenstand der Erfindung ist ein Verfahren zur Herstellung von Pankreatin-Pellets aus einer verformbaren Masse enthaltend Pankreatin-Pulver und Isopropanol oder Aceton umfassend die Schritte Mischen, Extrudieren, Zerteilen, Trocknen, Abrunden und magensaftresistentes Beschichten, wobei die verformbare Masse nur Pankreatin-Pulver und Isopropanol, Aceton oder ein Gemisch eines dieser Lösungsmittel mit flüssigen Chlorfluorkohlenwasserstoffen sowie gegebenenfalls ein Gleitmittel enthält.

Überraschenderweise wurde gefunden, daß nach dem erfindungsgemäßen Verfahren Pankreatin-Pellets mit hohen Pankreatin-Gehalten und guten mechanischen Eigenschaften unter wirtschaftlichen Bedingungen herstellbar sind.

Zur Durchführung des Verfahrens werden Pankreatin-Pulver und eines der vorstehend genannten Lösungsmittel miteinander in einem handelsüblichen Mischer zu einer verformbaren Masse vermischt. Als Pankreatin-Pulver wird handelsübliches, trockenes Material eingesetzt. Das Mischungsverhältnis vom Pankreatin-Pulver und Lösungsmittel ist derart gewählt, daß die resultierende Masse auf einer Strangpresse extrudierbar ist ; vorzugsweise werden pro 1 000 g Pankreatin-Pulver 70 ml bis 200 ml Lösungsmittel verwendet. Der zu verformenden Masse können außerdem bekannte, pharmazeutisch zulässige Gleitmittel zugesetzt werden.

Beim Extrudieren ist darauf zu achten, daß die auftretende Kompressionswärme die Enzyme nicht beschädigt. Zu diesem Zweck kann das zu extrudierende Gemisch z. B. vorgekühlt werden oder die Kompressionwärme kann durch zusätzliche Kühlvorrichtungen an der Strangpresse abgeführt werden. In einer andere. Verfahrensvariante wird dem Lösungsmittel Chlorfluorkohlenwasserstoffe als niedrigsiedende Komponente zugesetzt und der durch dessen Verdunstung bewirkte Energieeentzug zur Kühlung des Extrudats bzw. der Strangschnittlinge ausgenutzt. Die Chlorfluorkohlenwasserstoffe können in Anteilen von 0 bis 50, vorzugsweise 15 bis 35 Vol.-% des insgesamt verwendeten Lösungsmittels zugesetzt werden. Alle diese Maßnahmen sind für sich allein oder aber in beliebiger Kombination untereinander anwendbar.

Im erfindungsgemäßen Verfahren wird die verformbare Masse durch eine Lochplatte mit einer Matrizenbohrung oder mehreren solcher Bohrungen extrudiert. Besonders bevorzugt sind Matrizenbohrungen kreisförmigen Querschnitts. Der Durchmesser der Bohrungen ist prinzipiell unkritisch und wird nur von der späteren pharmazeutischen Verwendung der Pellets festgelegt. Sehr gute Erfahrungen wurden z. B. mit Durchmessern von 0,5 bis 4 mm, vorzugsweise 1 bis 2,5 mm gemacht, da Pellets dieses Durchmessers einerseits sehr gut weiterzuverarbeiten sind und andererseits bereits im Magen sehr gleichmäßig im Speisebrei verteilt werden können und daher

besonders gut und gleichmäßig wirksam sind.

Aus dem Extrudat werden nach an sich bekannten Methoden Strangschnittlinge hergestellt. Eine vorteilhafte Ausführung ist, Strangschnittlinge herzustellen, bei denen Durchmesser und Länge im Verhältnis von etwa 1 : 1 stehen, da derartige Strangschnittlinge leicht weiterzuverarbeiten sind.

Zur Weiterverarbeitung werden die Strangschnittlinge getrocknet und entweder direkt oder nach Abrundung zu im wesentlichen kugelförmigen Pellets weiterverarbeitet. Die Größe der abgerundeten Pellets bzw. die Abmessung der zur Abrundung einzusetzenden Strangschnittlinge wird u. a. von der beabsichtigten Darreichungsform bestimmt. Sollen die Pellets z. B. in Gelatinekapseln eingefüllt werden, so sind natürlich die Abmessungen mit der Kapselgröße abzustimmen.

Zur Abrundung können alle an sich bekannten Verfahren wie z. B. Umformung, Trommeln usw. eingesetzt werden. In einer besonderen Verfahrensvariante werden die Pellets nach dem Prinzip der Aufbaugranulation durch wechselweises oberflächliches Befeuchten und Einstreuen von Pankreatin-Pulver abgerundet.

Die abgerundeten Pellets werden zum Schutz der Enzyme vor der Einwirkung von Magensäure mit einem magensaftresistenten Überzug versehen, wobei es u. U. vorteilhaft sein kann, einen oder mehrere zusätzliche Überzüge z. B. zur Haftvermittlung, Glättung, etc. vorzusehen. Für den Fall, daß mehrere Überzüge aufgebracht werden, ist es vorteilhaft, nur den oder die ersten Überzüge aus einer Lösung oder Suspension des Überzugsmittels in enzymfreundlichem Lösungsmittel aufzubringen ; folgende Überzüge können dann aus wäßriger Phase verarbeitet werden.

Die nach dem erfindungsgemäßen Verfahren hergestellten Pellets können direkt als solche oder in anderer galenischer Form, z. B. in Form von Gelatinekapseln konfektioniert werden.

Folgende Vorteile zeichnen das erfindungsgemäße Verfahren aus :

a) es arbeitet umweltfreundlich und wirtschaftlich ;

b) der Durchmesser der Verfahrensprodukte kann in weitem Bereich variiert werden ;

c) die Verfahrensprodukte zeichnen sich durch Pankreatin-Gehalte über 80, vorzugsweise über 85 % aus, wodurch eine hohe Effizienz derartige Pellets enthaltender Arzneimittel erreicht wird ;

d) die Verfahrensprodukte verteilen sich bereits im Magen rasch und gleichmäßig im Speisebrei, ohne bereits dort zu zerfallen, da sie eine gute mechanische Festigkeit aufweisen ; auf diese Weise läßt sich eine optimale Wirkung der Pankreas-Enzyme erzielen.

Das erfindungsgemäße Verfahren wird durch die folgenden Beispiele nur erläutert und nicht beschränkt.

### Beispiel 1

4 400 g Pankreatin werden in einem Labormischer mit 150 g Magnesiumstearat gemischt und mit ca. 400 ml Isopropanol durchfeuchtet. Die Mischung wird auf einer Strangpresse mit Bohrungen von 1,6 mm lichter Weite komprimiert und auf Stranglänge von 1,5-1,7 mm geschnitten.

Nach Trocknung werden die zylindrischen Rohpellets in einem Dragierkessel durch wechselweises Auftragen einer 1 %igen isopropanolischen Lösung von Polyvinylpyrrolidon (Kollidon K 25) und Pankreatin zu sphärisch geformten Pellets ausgerundet. Hierfür werden ca. 1 l Lösung und 1 800 g Pankreatin benötigt.

Die runden Pellets werden durch Auftragen einer Lösung von 50 g Polyäthylenglykol 6 000 in 100 ml Isopropanol geglättet.

Nach Trocknung werden die Pellets in einem GLATT-Wirbelschichtgerät (WSGD 5) magensaftresistent überzogen. Hierzu werden für 6,3 kg Pankreatin-Pellets 7 200 g einer Lösung aus 590 g Hydroxypropylcellulosephthalat (Typ HP 55 ; Hersteller SHINETSU, Japan) und 150 g Dibutylphthalat in je 3 230 g Methylenchlorid und Isopropanol benötigt.

Ausbeute ca. 7 000 g magensaftresistente Pellets mit einem Durchmesser von 1,6 bis 2,0 mm und einem Gehalt von ca. 86 % Pankreatin.

### Beispiel 2

440 g Pankreatin werden in einem Labormischer mit einer Mischung aus 400 ml Isopropanol und 200 g sym. Tetrafluor-dichlor-äthan (Kaltron® 114 ; Hersteller Kali-Chemie AG) durchfeuchtet. Die Mischung wird auf einer Strangpresse mit Bohrungen von 1,6 mm lichter Weite komprimiert und auf eine Stranglänge von 1,5-1,7 mm geschnitten.

Nach Trocknung werden die zylindrischen Rohpellets in einem Dragierkessel durch wechselweises Auftragen einer 1 %igen Lösung von Polyvinylpyrrolidon (Kollidon K 25) und Pankreatin zu sphärisch geformten Pellets ausgerundet. Hierfür werden ca. 1 l Lösung und 1 800 g Pankreatin benötigt.

Die runden Pellets werden durch Auftragen einer Lösung von 30 g Kollidon K 25 in 100 ml Isopropanol geglättet.

Nach Trocknung werden die Pellets in einem GLATT-Wirbelschichtgerät (WSGD 5) magensaftresisten überzogen. Hierzu werden für 6,1 kg Pankreatin-Pellets 7 200 g einer Lösung aus 590 g HP 55 und 150 g Dibutylphthalat in je 3 230 g Methylenchlorid und Isopropanol benötigt.

Ausbeute ca. 6 800 g magensaftresistente Pellets mit einem Gehalt von ca. 89 % Pankreatin.

In einer Verfahrensvariante erfolgt die Ausrundung wie folgt :

250 g Polyäthylenglycol 6 000 werden bei 40-50 °C in 375 g Aceton gelöst. In der warmen Lösung werden 150 g Pankreatin-Pulver suspendiert. Diese Suspension wird bei 40 °C im Dragierkessel auf die ebenfalls auf 40 °C erwärmten Rohpellets (5 000 g) in mehreren Stufen aufgetragen. Der Kesselinhalt läuft bis zur Abkühlung auf Raumtemperatur. Es resultieren abgerundete Pellets, die bereits ohne Trocknung sehr gut handhabbar sind und die — wie beschrieben — mit weiteren Überzügen versehen werden.

**Patentansprüche**

1. Verfahren zur Herstellung von Pankreatin-Pellets aus einer verformbaren Masse enthaltend Pankreatin-Pulver und Isopropanol oder Aceton und umfassend die Schritte Mischen, Extrudieren, Zerteilen, Trocknen, Abrunden und magensaftresistentes Beschichten, dadurch gekennzeichnet, daß die verformbare Masse nur Pankreatin-Pulver und Isopropanol, Aceton oder ein Gemisch eines dieser Lösungsmittel mit flüssigen Chlorfluorkohlenwasserstoffen sowie gegebenenfalls ein Gleitmittel enthält.

2. Verfahren nach Anspruch 1, dadurch gekennzeichnet daß die verformbare Masse ein Gleitmittel, vorzugsweise · Magnesiumstearat enthält.

3. Verfahren nach einem der Ansprüche 1 oder 2, dadurch gekennzeichnet, daß man dem Lösungsmittel soviel Chlorfluorkohlenwasserstoff zusetzt, daß der Anteil dieses Zusatzes 0 bis 50, vorzugsweise 15 bis 25 vol.-% des insgesamt verwendeten Lösungsmittels beträgt.

4. Verfahren nach einem der Ansprüche 1 bis 3, dadurch gekennzeichnet, daß zur Herstellung der verformbaren Masse pro 1 000 g Pankreatin-Pulver 70 ml bis 200 ml Lösungsmittel verwendet werden.

5. Verfahren nach einem der Ansprüche 1 bis 4, dadurch gekennzeichnet, daß die durch Zerteilung der Extrudate erhaltenen Strangschnittlinge in an sich bekannten Vorrichtungen durch Aufbaugranulation unter Zusatz von Pankreatin-Pulver abgerundet werden.

6. Pankreatin-Pellets, hergestellt nach einem der vorstehenden Ansprüche, enthaltend mehr als 80 Gew.-%, vorzugsweise über 85 Gew.-% Pankreatin.

7. Arzneimittel, enthaltend nach dem Verfahren gemäß einem der Ansprüche 1 bis 5 hergestellte Pankreatin-Pellets.

**Claims**

1. Process for the preparation of pancreatin pellets from a mouldable mass containing pancreatin powder and isopropanol or acetone and comprising the steps of mixing, extruding, dividing, drying, rounding off and coating with a coating resistant to gastric juices, characterized in that the mouldable mass contains only pancreatin powder and isopropanol, acetone or of a mixture of one of these solvents with liquid chlorofluorohydrocarbons and, if required, a lubricant.

2. Process according to Claim 1, characterized in that the mouldable mass contains a lubricant, preferably magnesium stearate.

3. Process according to one of Claims 1 to 2, characterized in that sufficient chlorofluorohydrocarbon is added to the solvent for the proportion of this additive to be 0 to 50, preferably 15 to 25 % by volume of the total solvent used.

4. Process according to one of Claims 1 to 3, characterized in that 70 ml to 200 ml of solvent, per 1 000g of pancreatin powder, are used for the preparation of the mouldable mass.

5. Process according to one of Claims 1 to 4, characterized in that the short pieces obtained by division of the extrudate are rounded off in devices, which are in themselves known, by means of build-up granulation with the addition of pancreatin powder.

6. Pancreatin pellets prepared in accordance with one of the previous claims and containing more than 80 % by weight, preferably more than 85 % by weight, of pancreatin.

7. Medicaments containing pancreatin pellets which have been prepared by the process according to one of Claims 1 to 5.

**Revendications**

1. Procédé pour la fabrication de pellets de pancréatine à partir d'une matière moulable contenant de la poudre de pancréatine et de l'isopropanol ou de l'acétone, comprenant les opérations de malaxage, extrusion, division, séchage, arrondissage et enduction d'un revêtement résistant au suc gastrique, caractérisé en ce que la matière moulable ne contient que de la poudre de pancréatine et d'isopropanol, d'acétone ou d'un mélange d'un de ces solvants avec des hydrocarbures chlorés et fluorés liquides, ainsi qu'éventuellement d'un lubrifiant.

2. Procédé selon la revendication 1, caractérisé en ce que la matière moulable contient un lubrifiant, de préférence le stéarate de magnésium.

3. Procédé selon l'une des revendications 1 ou 2, caractérisé en ce qu'on ajoute au solvant autant d'hydrocarbure chloré et fluoré qu'il en faut pour que la proportion de cet adjuvant atteigne de 0 à 50, de préférence de 15 à 25 % en volume du solvant total utilisé.

4. Procédé selon l'une quelconque des revendications 1 à 3, caractérisé en ce que, pour la préparation de la matière moulable on utilise de 70 à 200 ml de solvant par 1 000 g de poudre de pancréatine.

5. Procédé selon l'une quelconque des revendications 1 à 4, caractérisé en ce que les ébauches de boudinage obtenues par division de l'extrudat sont arrondies dans des dispositifs connus en soi par granulation de formation en ajoutant de la poudre de pancréatine.

6. Pellets de pancréatine, fabriqués selon l'une des revendications précédentes, contenant plus de 80 % en poids, de préférence plus de 85 % en poids de pancréatine.

7. Médicament contenant des pellets de pancréatine fabriqués par le procédé selon l'une des revendications 1 à 5.